# EUROPEAN PATENT APPLICATION

(11) **EP 4 803 193 A1**
(43) Date of publication of application: **09.09.2026**
(21) Application number: 24885869.8
(22) Date of filing: 01.11.2024
(51) Int. Cl.: B01J 27/16, B01J 31/04, B01J 37/04, C07B 61/00, C07C 209/62, C07C 211/48

(54) **HYDROCRACKING CATALYST FOR CARBON-HETEROATOM BOND, METHOD FOR PRODUCING SAID HYDROCRACKING CATALYST, AND METHOD FOR HYDROCRACKING CARBON-HETEROATOM BOND**

(30) Priority: 02.11.2023 JP 2023188316
(71) Applicant: N.E. Chemcat Corporation, Tokyo 1055127 (JP)
(72) Inventor: KUWATA, Shoko, Tokyo 105-5127 (JP); MIZUSAKI, Tomoteru, Tokyo 105-5127 (JP)
(74) Representative: Regimbeau
(86) International application number: PCT/JP2024/039048
(87) International publication number: WO 2025/095105

(57) **Abstract**

[Problem to be Solved]

Provided is a hydrocracking reaction catalyst without any strong acid having a superior catalytic activity equal to or higher than that of conventional catalysts even in a case where the amount of palladium is reduced compared with those in conventional palladium catalysts.

[Solution]

A hydrocracking catalyst that hydrocracks a carbon-heteroatom bond of a substrate organic compound including the carbon-heteroatom bond, the hydrocracking catalyst including a carbon-based carrier at least supporting a palladium catalyst and at least one of a phosphoric acid compound and an acetic acid compound.

## Description

### Technical Field

The present patent application claims priority based on Japanese Patent Application No. 2023-188316 filed on November 2, 2023, the entire contents of which are incorporated herein by reference.

The present disclosure relates to a hydrocracking catalyst for a carbon-heteroatom bond, a method for producing the same, and a method for hydrocracking a carbon-heteroatom bond.

### Background Art

In the organic synthesis field, introduction and deprotection of a variety of protective groups are extremely important to synthesize intended compounds. In addition, a carbon-heteroatom bond is known as a structure in which introduction and deprotection of a variety of protective groups are easy to perform.

In a deprotection reaction of a protective group that has been once introduced (for example, a hydrocracking reaction of a benzyl group or the like), catalytic hydrogenation with a palladium/carbon catalyst is mainly used, but there is a problem in that when a large amount of palladium is not added as a catalyst, the reaction does not proceed toward a deprotection side. Therefore, regarding such a deprotection reaction, studies are underway for additional improvement since the cost, yield, or the like of the palladium catalyst is not sufficient.

For example, Patent Literature 1 proposes a method in which hydrogen is made to act in the presence of a palladium catalyst and an amine having one nitrogen atom, whereby the amine having one nitrogen atom modifies the palladium catalyst, and the modified palladium catalyst selectively hydrogenates a protective group (benzyl group).

In addition, Patent Literature 2 proposes a hydrocracking catalyst that brings a Bronsted acid catalyst and a palladium catalyst into contact with each other in a reactor.

### Citation List

### Patent Literature

Patent Literature 1: Japanese Patent Laid-Open No. 2017-197484
Patent Literature 2: International Publication No. WO 2021/251248

### Summary of Invention

However, the method in which a surface of a palladium catalyst is coated with an amine-based compound and the catalytic activity is adjusted, as proposed in Patent Literature 1, has a problem in that the catalytic activity deteriorates due to poisoning.

In addition, in the hydrocracking catalyst that has been proposed in Patent Literature 2, activated carbon treated with a strong acid, such as nitric acid or sulfuric acid, and having an acidic functional group imparted, which is used as a Bronsted acid catalyst, is mixed with a palladium carbon catalyst. However, the two catalysts have different deactivation periods and the strong acid is used during production, and therefore there have been problems related to productivity, such as management of heat of dilution generated by water in carbon wetted with water as well as treatment of waste liquid.

The present disclosure has been made in consideration of the above-described problems, and one object of the present disclosure is to provide a hydrocracking reaction catalyst without any strong acid having a superior catalytic activity equal to or higher than that of conventional catalysts even in a case where the amount of palladium is reduced compared with those in conventional palladium catalysts.

As a result of intensive studies, the present disclosers have found that when not only palladium but also a phosphoric acid compound or an acetic acid compound are supported on a carbon-based carrier, a hydrocracking reaction catalyst having a superior catalytic activity can be obtained without using a strong acid. The present disclosure is based on such knowledge.

One embodiment of the present disclosure provides a hydrocracking catalyst that hydrocracks a carbon-heteroatom bond of a substrate organic compound including the carbon-heteroatom bond, the hydrocracking catalyst including
a carbon-based carrier supporting at least
a palladium catalyst and
at least one of a phosphoric acid compound and an acetic acid compound.

According to the present disclosure, it is possible to provide a hydrocracking reaction catalyst without any strong acid having a superior catalytic activity equal to or higher than that of conventional catalysts even in a case where the amount of palladium is reduced compared with those in conventional palladium catalysts.

### Description of Embodiments

### [Hydrocracking catalyst]

In one embodiment of the present disclosure, in a hydrocracking catalyst that hydrocracks a carbon-heteroatom bond of a substrate organic compound including the carbon-heteroatom bond, the hydrocracking catalyst has one characteristic that it contains a carbon-based carrier at least supporting a palladium catalyst and at least one of a phosphoric acid compound and an acetic acid compound. Hereinafter, the hydrocracking catalyst of the present disclosure will be described in detail.

### (Palladium catalyst)

In the hydrocracking catalyst of the present disclosure, the above-described palladium catalyst is used as a hydrogenation catalyst. The palladium catalyst is suitable as long as it contains a palladium element as a catalyst species, and the catalyst may contain metallic palladium itself, or may contain a compound containing palladium (palladium compound). As the palladium catalyst, a well-known compound, such as particles or a variety of alloys, may also be used.

Examples of the palladium compound include tetrakis(triphenylphosphine)palladium, bis(triphenylphosphine)palladium chloride, palladium acetate, tris(dibenzylideneacetone)dipalladium, bis(dibenzalacetone)palladium, bis[4-(N,N-dimethylamino)phenyl]di-tert-butylphosphinepalladium chloride, bis(di-tert-butylprenylphosphine)palladium chloride, bis(di-tert-crotylphosphine)palladium dichloride, and the like.

The amount of the palladium catalyst in the hydrocracking catalyst is not particularly limited as long as the object of the present disclosure can be achieved and may be, for example, 1 to 20 parts by mass, preferably 1.5 to 18 parts by mass, more preferably 2 to 15 parts by mass, and still more preferably 3 to 13 parts by mass in 100 parts by mass of the hydrocracking catalyst (dry basis). In addition, the amount of the palladium element in the hydrocracking catalyst is not particularly limited as long as the object of the present disclosure can be achieved and may be, for example, 1 to 20 parts by mass, preferably 1.5 to 18 parts by mass, more preferably 2 to 15 parts by mass, and still more preferably 3 to 13 parts by mass in 100 parts by mass of the hydrocracking catalyst (dry basis). When the amount of the palladium catalyst or the palladium element is set within the above-described range, there is a case where the contact area between the palladium catalyst and the substrate organic compound is secured and the reactivity improves.

According to one embodiment of the present disclosure, the palladium catalyst is palladium itself (that is, a palladium element).

### (Phosphoric acid compound and acetic acid compound)

"Phosphoric acid compound" in the present disclosure means a compound having a hydroxyl group that bonds to at least a phosphorus atom. The phosphoric acid compound may be an organic phosphoric acid compound or an inorganic phosphoric acid compound.

The phosphoric acid compound is not limited thereto, and examples thereof include inorganic phosphoric acid compounds such as phosphoric acid (orthophosphoric acid), phosphorous acid, hypophosphorous acid, pyrophosphoric acid, metaphosphoric acid, and diphosphoric acid pentoxide; organic phosphoric acid compounds such as organic derivatives of phosphinic acid and phosphonic acid; and the like. According to one embodiment of the present disclosure, the phosphoric acid compound is an inorganic phosphoric acid compound and preferably an inorganic phosphoric acid compound having an acid dissociation constant pKa (H₂O) of one or higher. According to a preferable embodiment of the present disclosure, the phosphoric acid compound contains at least one selected from the group consisting of phosphoric acid (orthophosphoric acid), pyrophosphoric acid, metaphosphoric acid, and diphosphoric acid pentoxide. According to a more preferable embodiment of the present disclosure, the phosphoric acid compound contains at least phosphoric acid (orthophosphoric acid).

"Acetic acid compound" in the present disclosure means a compound having a structure "CR₃-C(O)OH" (in the formula, each "R" independently means hydrogen, a C₁₋₆ alkyl, or a halogen).

"C₁₋₆ alkyl" in the present disclosure means a linear or branched alkyl having 1 to 6 carbon atoms, and examples thereof include, but not limited to, methyl, ethyl, propyl, isopropyl, butyl, isobutyl, tert-butyl, and the like.

Examples of "halogen" in the present disclosure include fluorine, chlorine, bromine, iodine, and the like.

Examples of the acetic acid compound include, but not limited to, acetic acid, trifluoroacetic acid, monochloroacetic acid, dichloroacetic acid, dimethylacetic acid, and the like.

According to one embodiment of the present disclosure, the acetic acid compound is an acetic acid compound having an acid dissociation constant pKa (H₂O) of one or higher. According to a preferable embodiment of the present disclosure, the acetic acid compound contains at least one selected from the group consisting of acetic acid, monochloroacetic acid, dichloroacetic acid, and dimethylacetic acid. According to a more preferable embodiment of the present disclosure, the acetic acid compound contains at least acetic acid.

According to one embodiment of the present disclosure, the hydrocracking catalyst of the present disclosure contains at least a phosphoric acid compound. According to a preferable embodiment of the present disclosure, the hydrocracking catalyst of the present disclosure contains at least phosphoric acid (orthophosphoric acid). Advantageously, the phosphoric acid compound (preferably phosphoric acid) is less likely to cause either of any odor or any change over time due to decomposition during storage than other acids.

The amount of at least one of the phosphoric acid compound and the acetic acid compound in the hydrocracking catalyst is not particularly limited as long as the object of the present disclosure can be achieved and may be, for example, 1 to 20 parts by mass, preferably 1.5 to 15 parts by mass, more preferably 2 to 10 parts by mass, and still more preferably 3 to 8 parts by mass in 100 parts by mass of the hydrocracking catalyst (dry basis). In a case where the hydrocracking catalyst contains the phosphoric acid compound, the amount of the phosphorus element in the hydrocracking catalyst is not particularly limited as long as the object of the present disclosure can be achieved and may be, for example, 0.25 to 5 parts by mass, preferably 0.4 to 4 parts by mass, more preferably 0.5 to 2.5 parts by mass, and still more preferably 0.7 to 2 parts by mass in 100 parts by mass of the hydrocracking catalyst (dry basis).

The ratio between the palladium catalyst and at least one of the phosphoric acid compound and the acetic acid compound (the palladium catalyst/at least one of the phosphoric acid compound and the acetic acid compound) in the hydrocracking catalyst is not particularly limited as long as the object of the present disclosure can be achieved and may be, for example, 1:20 to 20:1, preferably 1:10 to 10:1, more preferably 1:5 to 5:1, and still more preferably 2:1 to 1:2 by these numbers of parts by mass of the palladium catalyst and at least one of the phosphoric acid compound and the acetic acid compound in the hydrocracking catalyst.

In a case where the hydrocracking catalyst contains the phosphoric acid compound, the ratio between the palladium element and the phosphoric element (the palladium element/the phosphoric element) in the hydrocracking catalyst is not particularly limited as long as the object of the present disclosure can be achieved and may be, for example, 1:10 to 40:1, preferably 1:5 to 20:1, more preferably 1:3 to 10:1, and still more preferably 1:1 to 5:1 by the numbers of parts by mass of the palladium element and the phosphoric element in the hydrocracking catalyst.

### (Carbon-based carrier)

In the hydrocracking catalyst of the present disclosure, at least the palladium catalyst and at least one of the phosphoric acid compound and the acetic acid compound are supported on a carbon-based carrier. The carbon-based carrier is not particularly limited as long as the carbon-based carrier is capable of supporting the palladium catalyst and at least one of the phosphoric acid compound and the acetic acid compound. Examples of the carbon-based carrier include, but not limited to, activated carbon, crushed activated carbon, mesoporous carbon, graphene, carbon nanotubes, glassy carbon (GC), fine carbon, carbon black, graphite, carbon fibers, and the like. According to one embodiment of the present disclosure, the carbon-based carrier contains at least one selected from the group consisting of activated carbon, mesoporous carbon, graphene, and a carbon nanotube.

The specific surface area of the carbon-based carrier is not particularly limited as long as the object of the present disclosure can be achieved. The specific surface area of the carbon-based carrier may be, for example, 1 m³/g or more, preferably 10 m³/g or more, more preferably 100 m³/g or more, and still more preferably 300 m³/g or more. In addition, the upper limit of the specific surface area of the carbon-based carrier may be, for example, 3000 m³/g or less, preferably 2000 m³/g or less, more preferably 1500 m³/g or less, and still more preferably 1000 m³/g or less.

The amount of the carbon-based carrier in the hydrocracking catalyst is not particularly limited as long as the object of the present disclosure can be achieved and may be, for example, 10 to 50 parts by mass, preferably 15 to 50 parts by mass, more preferably 25 to 50 parts by mass, and still more preferably 35 to 50 parts by mass in 100 parts by mass of the hydrocracking catalyst (dry basis).

The ratio between the palladium catalyst and the carbon-based carrier (the palladium catalyst/the carbon-based carrier) in the hydrocracking catalyst is not particularly limited as long as the object of the present disclosure can be achieved and may be, for example, 1:50 to 2:1, preferably 1:30 to 1:1, more preferably 1:20 to 1:3, and still more preferably 1:10 to 1:4 by the numbers of parts by mass of the palladium catalyst and the carbon-based carrier in the hydrocracking catalyst.

The ratio between at least one of the phosphoric acid compound and the acetic acid compound and the carbon-based carrier (at least one of the phosphoric acid compound and the acetic acid compound/the carbon-based carrier) in the hydrocracking catalyst is not particularly limited as long as the object of the present disclosure can be achieved and may be, for example, 1:50 to 2:1, preferably 1:30 to 1:1, more preferably 1:20 to 1:3, and still more preferably 1:10 to 1:4 by the numbers of parts by mass of at least one of the phosphoric acid compound and the acetic acid compound and the carbon-based carrier in the hydrocracking catalyst.

The hydrocracking catalyst of the present disclosure may contain moisture. The hydrocracking catalyst of the present disclosure containing moisture is advantageous in terms of preventing catalyst scattering and ignition during use. In a case where the hydrocracking catalyst of the present disclosure contains moisture, the amount of the moisture may be, for example, 5 to 70 parts by mass, more preferably 20 to 60 parts by mass, and still more preferably 45 to 55 parts by mass in 100 parts by mass of the hydrocracking catalyst.

In the hydrocracking catalyst of the present disclosure, other components may be supported on the carbon-based carrier as long as the object of the present disclosure is not impaired. Examples of the above-described other components include, but not limited to, noble metal elements other than palladium (for example, a platinum element, a ruthenium element, and the like), compounds thereof, compounds having a functional group such as a sulfo group, a carboxy group, or an amino group, and the like.

The hydrocracking catalyst of the present disclosure may contain other components (for example, other catalysts having a hydrocracking capability, a promotor that accelerates a hydrocracking reaction, and the like) aside from the carbon-based carrier at least supporting the palladium catalyst and at least one of the phosphoric acid compound and the acetic acid compound as long as the object of the present disclosure is not impaired.

### (Substrate organic compound including carbon-heteroatom bond)

The hydrocracking catalyst of the present disclosure is capable of hydrocracking a carbon-heteroatom bond of a substrate organic compound including the carbon-heteroatom bond.

"The carbon-heteroatom bond" used in the present disclosure means a bond formed between a carbon atom and an atom other than carbon atoms. Examples of the atom other than carbon atoms include, but not limited to, a nitrogen atom, an oxygen atom, a sulfur atom, a phosphorus atom, metal atoms, and the like, and a nitrogen atom and an oxygen atom are preferable. Therefore, examples of the carbon-heteroatom bond include a carbon-nitrogen bond, a carbon-oxygen bond, a carbonsulfur bond, a carbon-phosphorus bond, a carbon-metal atom bond, and the like. From the viewpoint of a likelihood of a hydrocracking reaction, the carbon-heteroatom bond is preferably a carbon-nitrogen bond or a carbon-oxygen bond and more preferably a carbon-nitrogen bond.

The carbon-heteroatom bond may be a single bond or may be a double bond or a triple bond. According to one embodiment of the present disclosure, the carbon-heteroatom bond is a single bond.

"The substrate organic compound including the carbon-heteroatom bond" (hereinafter also simply referred to as "the substrate organic compound") used in the present disclosure is not particularly limited as long as the substrate organic compound is an organic compound including at least one carbon-heteroatom bond in the molecule and capable of serving as a substrate for hydrocracking reactions. The substrate organic compound may be partially or fully chain-like (for example, linear or branched) or may be cyclic. In addition, in the substrate organic compound, a bond between carbon and carbon may be a single bond or may include one or a plurality of double bonds and/or triple bonds. In addition, in the substrate organic compound, one or a plurality of hydrogen atoms bonding to the carbon atom may be substituted with arbitrary substituents (for example, alkyl groups, cycloalkyl groups, alkenyl groups, cycloalkenyl groups, alkynyl groups, alkoxy groups, halogen groups, carboxyl groups, aldehyde groups, hydroxy groups, amino groups, or phenyl groups that may be substituted with one or a plurality of arbitrary substituents). In addition, one or a plurality of carbon atoms that configure the substrate organic compound may be substituted with heteroatoms (for example, oxygen atoms, nitrogen atoms, or sulfur atoms).

The hydrocracking catalyst of the present disclosure can be used for the purpose of a deprotection reaction of a protective group that protects a highly reactive portion in the molecule from reactions on other portions. Therefore, the substrate organic compound may be a compound in which a protective group coordinates to a heteroatom site in the carbon-heteroatom bond. Examples of "the protective group" in the present disclosure include, but not limited to, aralkyl groups (for example, a benzyl group, a 2-methylbenzyl group, a 3-methylbenzyl group, a 4-methylbenzyl group, a 2-chlorobenzyl group, a 3-chlorobenzyl group, a 4-chlorobenzyl group, a 2-bromobenzyl group, a 3-bromobenzyl group, a 4-bromobenzyl group, a 2-fluorobenzyl group, a 3-fluorobenzyl group, a 4-fluorobenzyl group, a 2-nitrobenzyl group, a 3-nitrobenzyl group, a 4-nitrobenzyl group, a 2-methoxybenzyl group, a 3-methoxybenzyl group, a 4-methoxybenzyl group, a diphenylmethyl group, and the like), aralkyloxycarbonyl groups (for example, aralkyloxycarbonyl groups such as a benzyloxycarbonyl group, a 2-nitrobenzyloxycarbonyl group, a 3-nitrobenzyloxycarbonyl group, a 4-nitrobenzyloxycarbonyl group, a 2-bromobenzyloxycarbonyl group, a 3-bromobenzyloxycarbonyl group, a 4-bromobenzyloxycarbonyl group, a 2-methoxybenzyloxycarbonyl group, a 3-methoxybenzyloxycarbonyl group, and a 4-methoxybenzyloxycarbonyl group, and the like), protective groups having an aromatic ring structure such as an alkyloxycarbonyl group (for example, a t-butyloxycarbonyl group, a t-amyloxycarbonyl group, and the like); trialkylsilylcarbonyl groups (for example, a trimethylsilyl group, a t-butyldimethylsilyl group, and the like), and the like. The protective group is preferably a protective group having an aromatic ring structure, more preferably an aralkyl group or an aralkyloxycarbonyl group, still more preferably a benzyl group, a 2-methoxybenzyl group, a 3-methoxybenzyl group, a 4-methoxybenzyl group, or a benzyloxycarbonyl group, and far still more preferably a benzyl group.

According to one embodiment of the present disclosure, the carbon-heteroatom bond is a bond between carbon adjacent to an aromatic ring and a heteroatom (preferably a bond between carbon adjacent to an aromatic ring and a nitrogen). "Carbon adjacent to an aromatic ring" in the present disclosure means a carbon atom apart from an atom that configures the aromatic ring (for example, a carbon atom, a nitrogen atom, a sulfur atom, or the like) by one atom (that is, the carbon atom that directly bonds to the atom that configures the aromatic ring), two atoms (that is, one different atom is present between the carbon atom and the atom that configures the aromatic ring), or three atoms (that is, two different atoms are present between the carbon atom and the atom that configures the aromatic ring). The carbon adjacent to an aromatic ring is preferably a carbon atom apart from the atom that configures the aromatic ring by one or two atoms and more preferably a carbon atom apart from the atom that configures the aromatic ring by one atom (that is, the carbon atom that directly bonds to the atom that configures the aromatic ring).

According to a preferable embodiment of the present disclosure, the carbon-heteroatom bond is a bond between a benzyl group and a nitrogen atom.

According to one embodiment of the present disclosure, the substrate organic compound is a compound in which a benzyl group coordinates to a nitrogen atom in an aniline structure. According to a preferable embodiment of the present disclosure, the substrate organic compound is N-benzyl-N-butyl-3-methylaniline.

### [Method for producing hydrocracking catalyst]

According to another embodiment of the present disclosure, provided is a method for producing a hydrocracking catalyst that hydrocracks a carbon-heteroatom bond of a substrate organic compound including the carbon-heteroatom bond, the method including
mixing a carbon-based carrier supporting a palladium catalyst and water, and
mixing a resulting mixture and a phosphoric acid compound or an acetic acid compound.

### (Mixing step of carbon-based carrier supporting palladium catalyst and water)

According to one embodiment of the present disclosure, in the above-described production method, a carbon-based carrier supporting a palladium catalyst and water are mixed together (also referred to as "first mixing step" in the present disclosure). In the first mixing step, a mixture (also referred to as "first mixture" in the present disclosure) may be obtained by mixing a carbon-based carrier supporting a palladium catalyst and water together using, for example, a desired stirring device (for example, a well-known stirring device). The first mixture may be in a slurry form.

The carbon-based carrier supporting a palladium catalyst that is used in the first mixing step may be a carrier obtained by supporting a palladium catalyst in a carbon-based carrier by a well-known method or may be a commercially available palladium-supported carbon-based carrier.

In the first mixing step, the mixing ratio between the carbon-based carrier supporting a palladium catalyst and water is not particularly limited and may be, for example, 10 to 10000 parts by mass, preferably 50 to 5000 parts by mass, more preferably 200 to 3000 parts by mass, and still more preferably 500 to 1000 parts by mass of water relative to 100 parts by mass of the carbon-based carrier supporting a palladium catalyst (dry weight).

In the first mixing step, conditions (the temperature, the time, the stirring rate, and the like) for mixing the carbon-based carrier supporting a palladium catalyst and water can be adjusted as appropriate by a person skilled in the art.

In the first mixing step, other components may be sequentially or simultaneously mixed as necessary to an extent that the object of the present disclosure is not impaired.

### (Mixing step of resulting mixture and at least one of phosphoric acid compound and acetic acid compound)

According to one embodiment of the present disclosure, in the above-described production method, the mixture (first mixture) obtained in the above-described first mixing step and a phosphoric acid compound or an acetic acid compound are mixed together (also referred to as "second mixing step" in the present disclosure). In the second mixing step, a mixture (also referred to as "second mixture" in the present disclosure) may be obtained by mixing the first mixture obtained in the above-described first mixing step (that is, a mixture containing the carbon-based carrier supporting a palladium catalyst and water) and at least one of a phosphoric acid compound and an acetic acid compound together using, for example, a desired stirring device (for example, a well-known stirring device).

In the second mixing step, the mixing ratio between the above-described first mixture and at least one of a phosphoric acid compound and an acetic acid compound is not particularly limited and may be, for example, 0.001 to 100 parts by mass, preferably 0.01 to 30 parts by mass, more preferably 0.1 to 10 parts by mass, and still more preferably 0.3 to 5 parts by mass of at least one of a phosphoric acid compound and an acetic acid compound relative to 100 parts by mass of the above-described first mixture.

In the second mixing step, conditions (the temperature, the time, the stirring rate, and the like) for mixing the above-described first mixture and at least one of a phosphoric acid compound and an acetic acid compound can be adjusted as appropriate by a person skilled in the art.

In the second mixing step, other components may be sequentially or simultaneously mixed as necessary to an extent that the object of the present disclosure is not impaired.

### (Filtering step)

According to one embodiment of the present disclosure, in the method for producing a hydrocracking catalyst, the second mixture is filtered (also referred to as "filtering step" in the present disclosure). When the filtering step is performed, an impurity in the hydrocracking catalyst (for example, at least one of the phosphoric acid compound and the acetic acid compound unreacted) or excess moisture can be removed, which is advantageous.

In the filtering step, the second mixture (or the second mixture that has been further treated) is filtered, and a filter residue is obtained. In the above-described filter residue, at least the carbon-based carrier at least supporting the palladium catalyst and at least one of the phosphoric acid compound and the acetic acid compound is contained.

In the filtering step, examples of a filtering method include, but not limited to, filter filtration, centrifugal filtration, and the like.

In the filtering step, conditions (the temperature, the time, the kind of a filter, and the like) during filtration can be adjusted as appropriate by a person skilled in the art.

### (Dehydrating step)

According to one embodiment of the present disclosure, the above-described second mixture (or the second mixture that has been further treated) or the above-described filter residue is dehydrated (also referred to as "dehydrating step" in the present disclosure). When the dehydrating step is performed, the amount of moisture in the hydrocracking catalyst can be made to be within a desired range, which is advantageous.

In the dehydrating step, the above-described second mixture (or the second mixture that has been further treated) or the above-described filter residue may be dehydrated by a desired method (for example, drying, centrifugation, or the like).

In the dehydrating step, conditions (the temperature, the time, the stirring rate, and the like) during dehydration can be adjusted as appropriate by a person skilled in the art.

The orders of the above-described filtering step and dehydrating step may be exchanged with each other as necessary.

To the method for producing a hydrocracking catalyst of the present disclosure, other steps aside from the above-described steps may be added as necessary.

### [Applications of hydrocracking catalyst]

The hydrocracking catalyst of the present disclosure can be used in the hydrocracking of a carbon-heteroatom bond of a substrate organic compound including the carbon-heteroatom bond. The hydrocracking catalyst of the present disclosure can be used for the purpose of a deprotection reaction of a protective group that protects a highly reactive portion in the molecule from reactions on other portions. Therefore, according to one embodiment of the present disclosure, the hydrocracking catalyst of the present disclosure is used to deprotect the above-described substrate compound.

According to another embodiment of the present disclosure, provided is a method for hydrocracking a carbon-heteroatom bond, the method including bringing the above-described substrate organic compound including the carbon-heteroatom bond, molecular hydrogen, and the above-described hydrocracking catalyst into contact with one another.

### (Contacting step of substrate organic compound, molecular hydrogen, and hydrocracking catalyst)

According to one embodiment of the present disclosure, in the above-described hydrocracking method, the above-described substrate organic compound including the carbon-heteroatom bond, molecular hydrogen, and the above-described hydrocracking catalyst are brought into contact with one another (also referred to as "contacting step" in the present disclosure). In the contacting step, it is possible to bring the above-described substrate organic compound including the carbon-heteroatom bond, molecular hydrogen, and the above-described hydrocracking catalyst into contact with one another in, for example, a desired reaction system (for example, a reaction container or the like) and to perform a hydrocracking reaction.

The ratio between the amounts of the hydrocracking catalyst and the substrate organic compound used is not particularly limited as long as a hydrocracking reaction can be caused in the ratio and may be, for example, 0.001 to 100 parts by mass, preferably 0.01 to 50 parts by mass, more preferably 0.1 to 20 parts by mass, and still more preferably 1 to 10 parts by mass of the hydrocracking catalyst relative to 100 parts by mass of the substrate organic compound. In addition, the ratio between the amounts of the hydrocracking catalyst and the substrate organic compound used may be, for example, 0.001 to 100 mol, preferably 0.01 to 10 mol, more preferably 0.05 to 5 mol, and still more preferably 0.1 to 3 mol of the hydrocracking catalyst relative to 1 mol of the substrate organic compound.

The molecular hydrogen needs to be present in a liquid phase or a gas phase in the reaction system (for example, a reaction container). The pressure of hydrogen that is supplied to the reaction system is not particularly limited as long as the hydrocracking reaction can progress and may be, for example, 0.01 to 1 MPa, preferably 0.05 to 0.8 MPa, and more preferably 0.1 to 0.6 MPa.

The above-described hydrocracking reaction may be performed in the presence of a desired reaction solvent. The above-described reaction solvent may be a solvent capable of dissolving at least a part of the above-described substrate organic compound and/or the above-described hydrocracking catalyst. In addition, the above-described reaction solvent is not particularly limited as long as the reaction solvent does not fully impair the hydrocracking reaction, and examples thereof include polar solvents such as ethers such as diethyl ether, t-butyl methyl ether, and tetrahydrofuran, alcohols such as 2-propanol, methanol, and ethanol, and N,N-dimethylformamide; non-polar solvents such as esters such as ethyl acetate and propyl acetate, chain hydrocarbons such as n-hexane and n-heptane, and cyclic hydrocarbons such as cyclohexane; and the like. The hydrocracking catalyst and/or hydrocracking method of the present disclosure is applicable to both polar solvents and non-polar solvents, which is advantageous.

The amount of the reaction solvent described above is not particularly limited as long as the hydrocracking reaction can progress in the amount and may be, for example, 1 to 10000 parts by mass, preferably 1 to 100 parts by mass, more preferably 10 to 50 parts by mass, and still more preferably 10 to 20 parts by mass relative to 100 parts by mass of the substrate organic compound from the viewpoint of performing a stable hydrocracking reaction.

The reaction temperature at the time of performing the hydrocracking reaction is not particularly limited as long as the hydrocracking reaction can progress at the temperature and may be, for example, -30°C to 80°C, preferably -15°C to 60°C, more preferably 0°C to 50°C, and still more preferably 10°C to 30°C. The above-described reaction temperature can be adjusted as appropriate by a person skilled in the art in consideration of the kind of the substrate organic compound used, the reaction time, or the like.

The reaction time at the time of performing the hydrocracking reaction is not particularly limited as long as the hydrocracking reaction can progress during the time and may be, for example, 0.1 to 48 hours, preferably 0.1 to 24 hours, and more preferably 0.1 to 10 hours. The above-described reaction time can be adjusted as appropriate by a person skilled in the art in consideration of the kind of the substrate organic compound used, the reaction temperature, or the like.

In the above-described hydrocracking reaction, in a case where bonds other than the intended carbon-heteroatom bond (for example, a bond of the aromatic ring, other double bonds, triple bonds, or the like present in the substrate organic compound) are hydrogenated, the amount of the hydrocracking catalyst, the amount of the molecular hydrogen, the pressure of hydrogen, the reaction temperature, the reaction time, and the like may be adjusted as appropriate, and the bonds other than the intended carbon-heteroatom bond may be protected with other protective groups (preferably protective groups that are not decomposed by the hydrocracking reaction by the hydrocracking catalyst of the present disclosure) or the like in advance.

### [Method for producing generated organic compound]

A generated organic compound in which at least one carbon-heteroatom bond of a substrate organic compound including the carbon-heteroatom bond has been hydrocracked can be produced by the above-described hydrocracking reaction. Therefore, according to still another embodiment of the present disclosure, provided is a method for producing a generated organic compound having at least one hydrocracked carbon-heteroatom bond described above, the method including bringing (preferably reacting) the above-described substrate organic compound including the carbon-heteroatom bond, the above-described molecular hydrogen, and the above-described hydrocracking catalyst into contact with one another in, for example, a reactor.

After a desired hydrocracking reaction ends, the above-described generated organic compound may be isolated from a post-reaction solution containing the above-described generated organic compound by, for example, a well-known method. Therefore, according to one embodiment of the present disclosure, the above-described production method may include isolating the generated organic compound obtained in the above-described reacting step. As an isolation method, for example, liquid-liquid separation, distillation, column chromatography, recrystallization, and the like may be used while the isolation method is not limited thereto.

The present disclosure includes the following:
[1] A hydrocracking catalyst that hydrocracks a carbon-heteroatom bond of a substrate organic compound including the carbon-heteroatom bond, the hydrocracking catalyst including:
   a carbon-based carrier at least supporting
   a palladium catalyst; and
   at least one of a phosphoric acid compound and an acetic acid compound.
[2] The hydrocracking catalyst according to [1], wherein a carbon-based carrier supporting at least a phosphoric acid compound is contained.
[3] The hydrocracking catalyst according to [1] or [2], wherein the phosphoric acid compound contains at least one selected from the group consisting of phosphoric acid (orthophosphoric acid), pyrophosphoric acid, metaphosphoric acid, and diphosphoric acid pentoxide.
[4] The hydrocracking catalyst according to any one of [1] to [3], wherein the phosphoric acid compound contains at least phosphoric acid (orthophosphoric acid).
[5] The hydrocracking catalyst according to [1], wherein the acetic acid compound contains at least one selected from the group consisting of acetic acid, trifluoroacetic acid, monochloroacetic acid, dichloroacetic acid, and dimethylacetic acid.
[6] The hydrocracking catalyst according to any one of [1] to [5], wherein the carbon-based carrier contains at least one selected from the group consisting of activated carbon, mesoporous carbon, graphene, and a carbon nanotube.
[7] The hydrocracking catalyst according to any one of [1] to [6], wherein in 100 parts by mass of the hydrocracking catalyst (dry basis), the palladium catalyst is 1 to 20 parts by mass, the phosphoric acid compound or the acetic acid compound is 1 to 20 parts by mass, and the carbon-based carrier is 10 to 50 parts by mass.
[8] The hydrocracking catalyst according to any one of [1] to [7], wherein in 100 parts by mass of the hydrocracking catalyst, a moisture content is 5 to 70 parts by mass.
[9] The hydrocracking catalyst according to any one of [1] to [8], wherein in 100 parts by mass of the hydrocracking catalyst (dry basis), a phosphorus element is 0.25 to 5 parts by mass.
[10] The hydrocracking catalyst according to any one of [1] to [9], wherein the carbon-heteroatom bond is a carbon-nitrogen bond.
[11] The hydrocracking catalyst according to [10], wherein the carbon-nitrogen bond is a bond between a carbon atom adjacent to an aromatic ring and a nitrogen atom.
[12] A method for producing a hydrocracking catalyst that hydrocracks a carbon-heteroatom bond of a substrate organic compound including the carbon-heteroatom bond, the method including:
   mixing a carbon-based carrier supporting a palladium catalyst and water; and
   mixing a resulting mixture and at least one of a phosphoric acid compound and an acetic acid compound.
[13] A method for producing a generated organic compound having at least one hydrocracked carbon-heteroatom bond, the method including:
   bringing a substrate organic compound including the carbon-heteroatom bond, molecular hydrogen, and a hydrocracking catalyst into contact with one another in a reactor,
   wherein the hydrocracking catalyst contains a carbon-based carrier at least supporting a palladium catalyst and at least one of a phosphoric acid compound and an acetic acid compound.

### Examples

Hereinafter, the hydrocracking catalyst of the present disclosure will be described in more detail using examples. The following examples, however, are not intended to limit the hydrocracking catalyst of the present disclosure by any means. Unless particularly otherwise described, percentages or ratios described in the present specification are mass-based. In addition, unless particularly otherwise described, units or measuring methods described in the present specification are based on the regulations of Japanese Industrial Standards (JIS).

### [Example1: Phosphoric acid-supported 5% Pd/C catalyst]

120 g (dry weight) of 5% Pd/C (manufactured by N.E. CHEMCAT CORPORATION, trade name: Pd/C Type PE (Pd 5%) (wetted with water)) and 960 mL of pure water were mixed together and stirred for five minutes to obtain a Pd/C aqueous solution. Next, the above-described Pd/C aqueous solution and 10.3 mL of a 85% phosphoric acid aqueous solution were mixed together, stirred for 30 minutes, then, filtered, and dehydrated, thereby obtaining a hydrocracking catalyst (phosphoric acid-supported 5% Pd/C catalyst) of Example 1.

As a result of evaluating the 5% Pd/C used as a raw material by elemental analysis, since the amount of a palladium element in a carrier was 4.73%, the amount of a carbon-based carrier in the 5% Pd/C was estimated to be 95.27% (dry basis content).

The moisture content of the resulting hydrocracking catalyst of Example 1 was 51.34%. In addition, as a result of evaluating the hydrocracking catalyst of Example 1 by elemental analysis, the amount of a phosphorus element in the carrier was 1.63% (dry basis content), and it was estimated that 5.4 g of phosphoric acid was supported per 100 g of the hydrocracking catalyst (dry basis). Therefore, the hydrocracking catalyst of Example 1 was estimated to contain approximately 5% of a palladium catalyst (palladium element) in terms of dry basis and approximately 44% of the carbon-based carrier in terms of dry basis.

### [Example 2: Phosphoric acid-supported 10% Pd/C catalyst]

5.0 g (dry weight) of 10% Pd/C (manufactured by N.E. CHEMCAT CORPORATION, trade name: P-10D (wetted with water)) and 40 mL of pure water were mixed together and stirred for five minutes to obtain a Pd/C aqueous solution. Next, the above-described Pd/C aqueous solution and 0.43 mL of 85% phosphoric acid were mixed together, stirred for 30 minutes, then, filtered, and dehydrated, thereby obtaining a hydrocracking catalyst (phosphoric acid-supported 10% Pd/C catalyst) of Example 2.

The moisture content of the resulting hydrocracking catalyst of Example 2 was 50%.

### [Example 3: Acetic acid-supported 10% Pd/C catalyst]

5.0 g (dry weight) of 10% Pd/C (manufactured by N.E. CHEMCAT CORPORATION, trade name: P-10D (wetted with water)) and 40 mL of pure water were mixed together and stirred for five minutes to obtain a Pd/C aqueous solution. Next, the above-described Pd/C aqueous solution and 0.43 mL of acetic acid were mixed together, stirred for 30 minutes, then, filtered, and dehydrated, thereby obtaining a hydrocracking catalyst (acetic acid-supported 10% Pd/C catalyst) of Example 3.

The moisture content of the resulting hydrocracking catalyst of Example 3 was 50%.

### [Reference Example 1: 5% Pd/C catalyst]

5% Pd/C (manufactured by N.E. CHEMCAT CORPORATION, trade name: Pd/C Type PE (Pd 5%) (wetted with water)) was used as a hydrocracking catalyst of Reference Example 1.

### [Reference Examples 2 and 3: 10% Pd/C catalysts]

10% Pd/C (manufactured by N.E. CHEMCAT CORPORATION, trade name: P-10D (wetted with water)) was used as hydrocracking catalysts of Reference Examples 2 and 3.

### [Reference Example 4: Mixture of 10% Pd/C catalyst and sulfo group-imparted activated carbon]

20 mg of 10% Pd/C (manufactured by N.E. CHEMCAT CORPORATION, trade name: P-10D (wetted with water)) and 30 mg of sulfo group-imparted activated carbon, which was activated carbon having an acidic group (manufactured by Futamura Chemical Co., Ltd., trade name: Taiko CP, catalog value: 60% to 70% of moisture, particle size: less than 0.18 mm, specific surface area: less than 50 m²/g, sulfo group amount: 2.0 to 2.5 mmol/g), were mixed together to obtain a hydrocracking catalyst of Reference Example 4.

The content of Pd in the resulting hydrocracking catalyst of Reference Example 4 was approximately 5% (content in terms of dry basis).

### [Test Example 1: Hydrocracking reaction (debenzylation) 1/reaction in ethyl acetate]

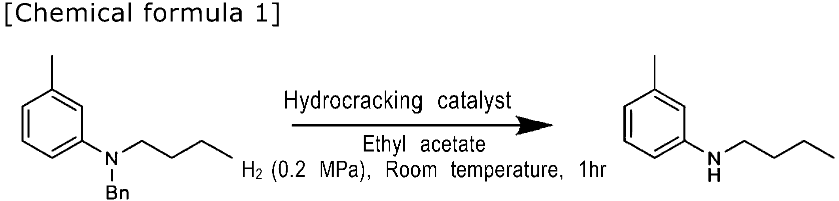

0.8 mmol of N-benzyl-N-butyl-3-methylaniline, which was a substrate organic compound, and the hydrocracking catalyst of each of Example 1 and Reference Examples 1 to 4 were mixed together in an ethyl acetate solvent, and debenzylation was performed while the components were stirred at a hydrogen pressure of 0.2 MPa and room temperature (23°C) for one hour. A resulting post-reaction solution was analyzed by gas chromatography (device: GC-2010 manufactured by Shimadzu Corporation, column: DB-1 30.0 m, mobile phase: He), and a conversion rate was calculated from the amount of the substrate organic compound decreased. The results are shown in Table 1. Since only a peak of N-butyl-m-toluidine was shown from the resulting post-reaction solution, debenzylation was considered to have occurred. Moreover, in Table 1, weight ratios between the substrate organic compound and Pd were shown as catalyst amounts (wt%).

**[Table 1]**

| | Catalyst amount (wt%) | Substrate | Reactant | Conversion rate |
|---|---|---|---|---|
| Example 1 (Phosphoric acid-supported 5% Pd/C) | 5 | 0 | 100 | 100% |
| Reference Example 1 (5%Pd/C) | 5 | 34 | 66 | 66% |
| Reference Example 2 (10%Pd/C) | 5 | 7 | 93 | 93% |
| Reference Example 3 (10%Pd/C) | 2.5 | 34 | 66 | 66% |
| Reference Example 4 (10%Pd/C+SO₃H-C) | 5 | 9 | 91 | 91% |

### [Test Example 2: Hydrocracking reaction (debenzylation) 2/reaction in cyclohexane]

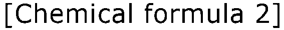

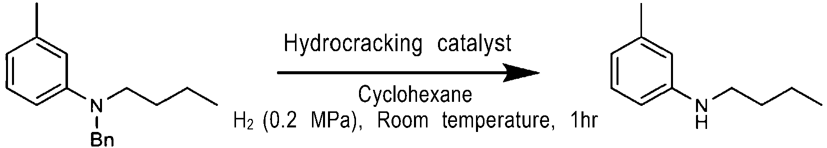

0.8 mmol of N-benzyl-N-butyl-3-methylaniline, which was a substrate organic compound, and the hydrocracking catalyst obtained in each of Examples 2 and 3 and Reference Example 2 were mixed together in a cyclohexane solvent, and debenzylation was performed while the components were stirred at a hydrogen pressure of 0.2 MPa and room temperature (set to 23°C) for one hour. A resulting post-reaction solution was analyzed by gas chromatography (under the same conditions as in Test Example 1), and a conversion rate was calculated from the amount of the substrate organic compound decreased. The results are shown in Table 2. Moreover, weight ratios between the substrate organic compound and Pd were shown as catalyst amounts (wt%) in Table 2.

**[Table 2]**

| | Catalyst amount (wt%) | Substrate | Reactant | Conversion rate |
|---|---|---|---|---|
| Example 2 (Phosphoric acid-supported 10% Pd/C) | 5wt% | 13 | 87 | 87% |
| Example 3 (Acetic acid-supported 10% Pd/C) | 5wt% | 7 | 93 | 93% |
| Reference Example 2 (10%Pd/C) | 5wt% | 86 | 14 | 14% |

### [Test Example 3: Hydrocracking reaction (debenzylation) 3/reactions in various solvents]

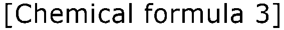

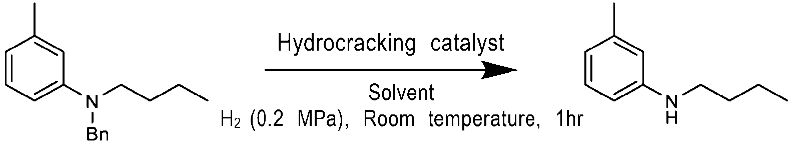

2.5 mmol of N-benzyl-N-butyl-3-methylaniline, which was a substrate organic compound, and the hydrocracking catalyst obtained in Example 1 were mixed together in solvents shown in Table 3, and debenzylation was performed while the components were stirred at a hydrogen pressure of 0.2 MPa and room temperature (set to 23°C) for one hour. Resulting post-reaction solutions were analyzed by gas chromatography (under the same conditions as in Test Example 1), and conversion rates were calculated from the amounts of the substrate organic compound decreased. The results are shown in Table 3. Moreover, weight ratios between the substrate organic compound and Pd were shown as catalyst amounts (wt%) in Table 3.

**[Table 3]**

| | Catalyst amount (wt%) | Solvent | Substrate | Reactant | Conversion rate |
|---|---|---|---|---|---|
| Example 1 (Phosphoric acid-supported 5% Pd/C) | 5 | DMF | 0 | 100 | 100% |
| Example 1 (Phosphoric acid-supported 5% Pd/C) | 5 | THF | 6 | 94 | 94% |
| Example 1 (Phosphoric acid-supported 5% Pd/C) | 5 | MeOH | 0 | 100 | 100% |
| Example 1 (Phosphoric acid-supported 5% Pd/C) | 5 | EtOH | 0 | 100 | 100% |
| Example 1 (Phosphoric acid-supported 5% Pd/C) | 5 | 2-PrOH | 0 | 100 | 100% |

| | | | | | |
|---|---|---|---|---|---|
| *: DMF: N,N-Dimethylformamide, THF: Tetrahydrofuran, MeOH: Methanol, EtOH: Ethanol, 2-PrOH: 2-Propanol | | | | | |

### [Test Examples 4 to 6: Hydrocracking reaction (debenzylation) 4/reactions in various substrates]

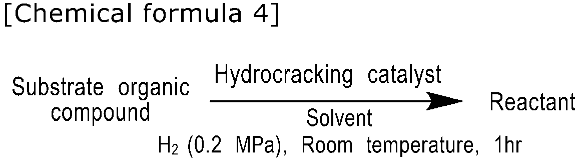

2.5 mmol of each of substrate organic compounds shown in Table 4 and the hydrocracking catalyst obtained in Example 1 were mixed together in each of solvents shown in Table 4, and debenzylation was performed while the components were stirred at a hydrogen pressure of 0.2 MPa and room temperature (set to 23°C) for one hour. Resulting post-reaction solutions were analyzed by gas chromatography (under the same conditions as in Test Example 1), and conversion rates were calculated from the amounts of the substrate organic compound decreased. The results are shown in Table 5. Moreover, weight ratios between the substrate organic compound and Pd were shown as catalyst amounts (wt%) in Table 5.

**[Table 4]**

| | Substrate | Reactant | Solvent |
|---|---|---|---|
| Test Example 4 | 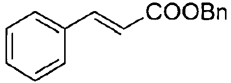 | 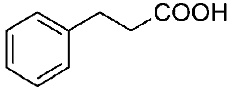 | Cyclohexane |
| Test Example 5 | 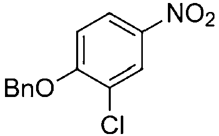 | 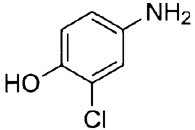 | DMF |
| Test Example 6 | 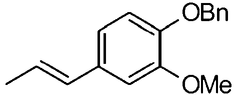 | 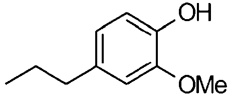 | AcOEt |

| | | | |
|---|---|---|---|
| *: DMF: N,N-Dimethylformamide, AcOEt: Ethyl acetate | | | |

**[Table 5]**

| | Catalyst amount (wt%) | Substrate | Reactant | Conversion rate |
|---|---|---|---|---|
| Test Example 4 | 5 | 0 | 100 | 100% |
| Test Example 5 | 5 | 0 | 100 | 100% |
| Test Example 6 | 5 | 0 | 100 | 100% |

As is clear from the results of Test Example 1, the hydrocracking catalyst of the present disclosure exhibited a superior catalytic activity compared with that of the conventional hydrocracking catalyst supporting the same amount of palladium in a polar solvent (for example, ethyl acetate) (for example, Reference Example 1). Furthermore, the hydrocracking catalyst of the present disclosure exhibited a superior catalytic activity equal to or high than that of the conventional hydrocracking catalyst supporting approximately twice the amount of palladium (for example, Reference Example 2). In addition, the hydrocracking catalyst of the present disclosure exhibited a superior catalytic activity equal to or higher than the hydrocracking catalyst of Reference Example 4 (corresponding to the hydrocracking catalyst described in Patent Literature 2).

In the hydrocracking catalyst described in Patent Literature 2, since the two catalysts have different deactivation periods and a strong acid is used during production, there have been problems related to productivity, such as management of heat of dilution generated by water in carbon wetted with water as well as treatment of waste liquid. On the other hand, in the hydrocracking catalyst of the present disclosure, since two catalysts are not always required and a strong acid is not always required, there is no such a problem, which is advantageous. Furthermore, it is especially advantageous that the hydrocracking catalyst of the present disclosure exhibits a superior catalytic activity equal to or higher than that of the hydrocracking catalyst described in Patent Literature 2 regardless of the fact that two catalysts are not always required and a strong acid is also not always required.

As is clear from the results of Test Example 2, the hydrocracking catalysts of the present disclosure exhibited a superior catalytic activity even in a non-polar solvent (for example, cyclohexane) compared with the conventional hydrocracking catalyst supporting the same amount of palladium (for example, Reference Example 2). Furthermore, as is clear from the results of Test Example 3, the hydrocracking catalysts of the present disclosure exhibited a superior catalytic activity even in a case where a wide range of solvents were used. Therefore, the hydrocracking catalyst of the present disclosure is capable of exhibiting a catalytic activity even in a wide range of solvents, which is advantageous.

As is clear from the results of Test Examples 4 to 6, the hydrocracking catalysts of the present disclosure exhibited a catalytic activity even with respect to a wide range of substrate organic compounds. Particularly, in Test Examples 4 and 6, the hydrocracking catalysts of the present disclosure are considered to be capable of not only debenzylation but also hydrocracking of a double bond (a double bond between carbon and carbon) in a side chain. Therefore, the hydrocracking catalyst of the present disclosure is capable of hydrocracking not only a carbon-heteroatom bond but also an unsaturated bond between carbon and carbon, which is advantageous.

The hydrocracking catalyst of the present disclosure exhibits an excellent hydrocracking reaction with respect to a carbon-heteroatom bond. While not bound by any theory, it is considered that, for example, in the case of using N-benzyl-N-butyl-3-methylaniline as a substrate organic compound, the phosphoric acid compound or the acetic acid compound (preferably phosphoric acid or acetic acid) donates a proton to a nitrogen position to which a benzyl group coordinates, after that, the palladium catalyst comes into contact with the position, and hydrocracking (debenzylation) occurs. Particularly, it is considered that since a proton donating reaction may make the benzyl group unstable, it is possible to cause hydrocracking by palladium with a smaller amount of activation energy than ever, and consequently, a superior catalytic activity equal to or higher than that of conventional catalysts can be obtained even when the amount of palladium is small compared with those in conventional catalysts.

Conventionally, it has been considered that in the above-described reaction, a functional group in some form needs to be imparted onto a carrier due to a proper distance from a catalyst; however, surprisingly, it is considered that the above-described effect can be obtained simply by supporting a phosphoric acid compound or an acetic acid compound (preferably phosphoric acid or acetic acid) on a carrier.

In addition, it is known that an aromatic ring in a benzyl group or the like easily radicalizes a heteroatom in a coordination site due to a resonance structure, and it is considered that regardless of the embodiments of the present disclosure, a particularly excellent hydrocracking reaction occurs in an organic compound having a protective group including an aromatic ring coordinated to a heteroatom.

## Claims

1. A hydrocracking catalyst that hydrocracks a carbon-heteroatom bond of a substrate organic compound including the carbon-heteroatom bond, the hydrocracking catalyst comprising:
a carbon-based carrier at least supporting
a palladium catalyst; and
at least one of a phosphoric acid compound and an acetic acid compound.

2. The hydrocracking catalyst according to Claim 1, wherein a carbon-based carrier supporting at least a phosphoric acid compound is comprised.

3. The hydrocracking catalyst according to Claim 1, wherein the phosphoric acid compound comprises at least one selected from the group consisting of phosphoric acid (orthophosphoric acid), pyrophosphoric acid, metaphosphoric acid, and diphosphoric acid pentoxide.

4. The hydrocracking catalyst according to Claim 1, wherein the phosphoric acid compound comprises at least phosphoric acid (orthophosphoric acid).

5. The hydrocracking catalyst according to Claim 1, wherein the acetic acid compound comprises at least one selected from the group consisting of acetic acid, trifluoroacetic acid, monochloroacetic acid, dichloroacetic acid, and dimethylacetic acid.

6. The hydrocracking catalyst according to Claim 1, wherein the carbon-based carrier comprises at least one selected from the group consisting of activated carbon, mesoporous carbon, graphene, and a carbon nanotube.

7. The hydrocracking catalyst according to Claim 1, wherein in 100 parts by mass of the hydrocracking catalyst (dry basis), the palladium catalyst is 1 to 20 parts by mass, the phosphoric acid compound or the acetic acid compound is 1 to 20 parts by mass, and the carbon-based carrier is 10 to 50 parts by mass.

8. The hydrocracking catalyst according to Claim 1, wherein in 100 parts by mass of the hydrocracking catalyst, a water content is 5 to 70 parts by mass.

9. The hydrocracking catalyst according to Claim 1, wherein in 100 parts by mass of the hydrocracking catalyst (dry basis), a phosphorus element is 0.25 to 5 parts by mass.

10. The hydrocracking catalyst according to any one of Claims 1 to 9, wherein the carbon-heteroatom bond is a carbon-nitrogen bond.

11. The hydrocracking catalyst according to Claim 10, wherein the carbon-nitrogen bond is a bond between a carbon atom adjacent to an aromatic ring and a nitrogen atom.

12. A method for producing a hydrocracking catalyst that hydrocracks a carbon-heteroatom bond of a substrate organic compound including the carbon-heteroatom bond, the method comprising:
mixing a carbon-based carrier supporting a palladium catalyst and water; and
mixing a resulting mixture and at least one of a phosphoric acid compound and an acetic acid compound.

13. A method for producing a generated organic compound having at least one hydrocracked carbon-heteroatom bond, the method comprising:
bringing a substrate organic compound including the carbon-heteroatom bond, molecular hydrogen, and a hydrocracking catalyst into contact with one another in a reactor,
wherein the hydrocracking catalyst comprises a carbon-based carrier at least supporting a palladium catalyst and at least one of a phosphoric acid compound and an acetic acid compound.
